(19)

Europäisches
Patentamt

European
Patent Office

Office européen
des brevets

(11) **EP 4 628 142 A1**

(12) **EUROPEAN PATENT APPLICATION**
published in accordance with Art. 153(4) EPC

(43) Date of publication:
**08.10.2025 Bulletin 2025/41**

(21) Application number: **23897889.4**

(22) Date of filing: **30.11.2023**

(51) International Patent Classification (IPC):
**A61M 37/00** (2006.01)

(52) Cooperative Patent Classification (CPC):
**A61K 9/00; A61K 9/70; A61M 37/00**

(86) International application number:
**PCT/JP2023/043025**

(87) International publication number:
**WO 2024/117243 (06.06.2024 Gazette 2024/23)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **02.12.2022 JP 2022193594**

(71) Applicant: **Kao Corporation**
**Chuo-ku,**
**Tokyo 103-8210 (JP)**

(72) Inventors:
• **NIITSU, Takatoshi**
**Tokyo 103-8210 (JP)**
• **NISHIMURA, Tomoya**
**Tokyo 103-8210 (JP)**
• **HARIU, Wataru**
**Tokyo 103-8210 (JP)**
• **ONISHI, Shintaro**
**Tokyo 103-8210 (JP)**
• **KOIKE, Natsumi**
**Tokyo 103-8210 (JP)**

(74) Representative: **Jones, Nicholas Andrew**
**Withers & Rogers LLP**
**2 London Bridge**
**London SE1 9RA (GB)**

(54) **INJECTION NEEDLE**

(57) An injection needle includes first and second projections (11, 12); wherein the first projection and the second projection each are fine and protrude from a sheet base portion (2), the first projection (11) has a conical shape and includes an opening (11a) in a distal end portion thereof, the second projection (12) does not include an opening in a distal end portion thereof, and the first projection and the second projection each have a protruding height of 5000 μm or less. The injection needle includes a first region (R1) and a second region (R2) in plan view; wherein first region (R1) includes one or two or more first projections (11); the second region (R2) includes two or more second projections (12); and the second region (R2) surrounds the first region (R1) or the second regions (R2) sandwich the first region (R1). A distance (D1), which is the shortest distance among distances between the first projection (11) and the second projections (12), is from 2.0 mm to 10 mm.

Fig. 1

EP 4 628 142 A1

## Description

Technical Field

The present invention relates to an injection needle.

Background Art

In recent years, intradermal administration of a liquid such as a drug by an injection needle including a fine needle-like projection called a microneedle or the like has attracted attention in the medical field, the cosmetic field, and the like. With this injection needle, it is possible to inject a liquid into a body by inserting the microneedle into a relatively shallow layer in the skin such as the stratum corneum, significantly reducing pain felt by a subject as compared with a normal injector. As a result, the injection needle has attracted attention as a minimally invasive liquid administration means.

Patent Literature 1 describes a hub assembly including a columnar limiter from which a microneedle protrudes, and a stabilization ring surrounding the microneedle and the limiter. The limiter is for controlling the insertion depth of the microneedle into the skin. The stabilization ring is for suppressing deformation of the skin when the microneedle is inserted into the skin. The hub assembly includes an attachment mechanism and is fixed to a delivery device of the drug via the attachment mechanism.

Patent Literature 2 describes a microneedle sheet including a plurality of needles having a minute size and protruding from the surface of the microneedle sheet. This document discloses that the needles include penetrating needles each including a hole penetrating from the front surface side to the rear surface side and dummy needles each including no penetrating hole.

Citation List

Patent Literature

Patent Literature 1: WO 2007/061972 A2
Patent Literature 2: WO 2021/177317

Summary of Invention

The present invention relates to an injection needle.

In one embodiment, preferably the injection needle includes a first projection and a second projection, each being fine and protruding from a sheet base portion.

In one embodiment, preferably the first projection has a conical shape and includes an opening in a distal end portion thereof.

In one embodiment, preferably the second projection does not include an opening in a distal end portion thereof.

In one embodiment, preferably the injection needle includes a first region and a second region, in plan view; wherein the first region includes one or two or more first projections; the second region includes two or more second projections; and the second region surrounds or sandwiches the first region.

In one embodiment, preferably a shortest distance among distances between the first projection and the second projections, D1, is from 2.0 mm to 10 mm.

Brief Description of Drawings

[Fig. 1] Fig. 1 is a perspective view of an injection needle according to a preferred embodiment of the present invention.
[Fig. 2] Fig. 2 is a plan view of the injection needle illustrated in Fig. 1.
[Fig. 3] Fig. 3(a) is a cross-sectional view taken along line A-A in Fig. 2. Fig. 3(b) is a cross-sectional view taken along line B-B in Fig. 2. Fig. 3(c) is a cross-sectional view taken along line C-C in Fig. 2.
[Fig. 4] Fig. 4 is a plan view schematically illustrating an arrangement pattern of projections in the injection needle illustrated in Fig. 1.
[Fig. 5] Fig. 5 is a view schematically illustrating a state of use of the injection needle illustrated in Fig. 1.
[Fig. 6] Figs. 6(a) to (d) are plan views illustrating other preferred embodiments of the present invention and correspond to Fig. 4.

[Fig. 7] Fig. 7 is a cross-sectional view illustrating a second projection according to another preferred embodiment of the present invention.

[Fig. 8] Figs. 8(a) and (b) are views schematically illustrating a state of use of an injection needle in the related art.

[Fig. 9] Fig. 9(a) is a plan view schematically illustrating an arrangement pattern of projections in an injection needle of comparative example 1, and Fig. 9(b) is a plan view schematically illustrating an arrangement pattern of projections in an injection needle of comparative example 2.

[Fig. 10] Fig. 10 is a plan view schematically illustrating an arrangement of projections of an injection needle used for evaluation of a relationship between liquid volume and a radius of a wheal.

[Fig. 11] Fig. 11 is a plan view schematically illustrating a major axis and a minor axis of the wheal measured in the evaluation of the relationship between the liquid volume and the radius of the wheal.

[Fig. 12] Fig. 12 is a graph showing evaluation results of the relationship between the liquid volume and the radius of the wheal.

Description of Embodiments

Intradermal injection is attracting attention as an injection method for enabling an antigen to be efficiently administered. The World Health Organization and others have reported that intradermal injection can reduce an amount of vaccine required for one inoculation to one-fifth as compared with intramuscular injection or subcutaneous injection. However, it is known that considerable training is required to master the procedure of intradermal administration with injection needles in the related art. To facilitate intradermal administration, intradermal administration of a liquid such as a drug by an injection needle including a fine needle-like projection called a microneedle or the like has attracted attention. However, in use of microneedles in the related art, it may be difficult to appropriately inject a liquid such as a drug into the skin, due to a wheal formed when the liquid is injected into the skin. This point will be described in detail below.

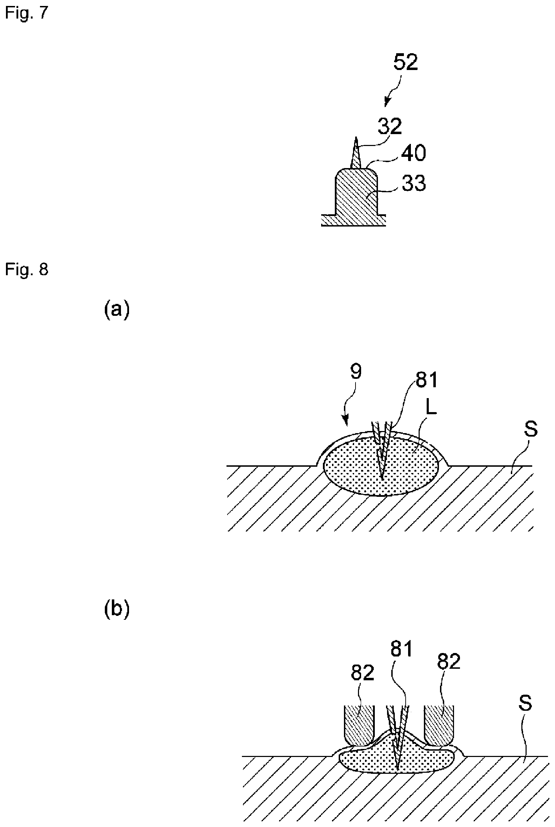

The microneedle is inserted into the skin to inject a liquid into the skin. When a liquid is injected into the skin, the injected liquid causes rising of the skin and thus the formation of a wheal (see Fig. 8(a)). In a case where the injection needle includes projections 82 in addition to a microneedle 81 for injecting the liquid, such as the stabilization ring in Patent Literature 1 and the dummy needle in Patent Literature 2, these projections 82 may suppress the rising of the skin (see Fig. 8(b)). This may lead to failure in the liquid injection, and specifically, may lead to liquid leakage or difficulty in allowing the liquid to enter into the body, when the injection volume is large, for example. Thus, in Patent Literature 1 and 2, injection of the liquid may be difficult.

The present invention relates to an injection needle that less causes suppression of rising of the skin by the injected liquid to facilitate efficient injection of liquid, when the liquid is injected into the skin by the injection needle.

The present invention will now be described with reference to preferred embodiments.

An injection needle 1 of the present invention includes projections protruding from a sheet base portion 2. The sheet base portion 2 is a sheet-like portion connecting the projections. In a case where the injection needle 1 is provided by forming the projections on a base material sheet, the sheet base portion 2 is a portion other than the projections in the injection needle 1.

The injection needle 1 typically has a flat sheet-like shape as a whole, and includes the sheet base portion 2 and multiple ef-the projections protruding from one side of the sheet base portion 2.

The injection needle 1 typically includes a projection arrangement region in which the projections are arrayed in a dispersed state in a planar direction.

The projection arrangement region typically includes a first region R1 and a second region R2, which will be described later.

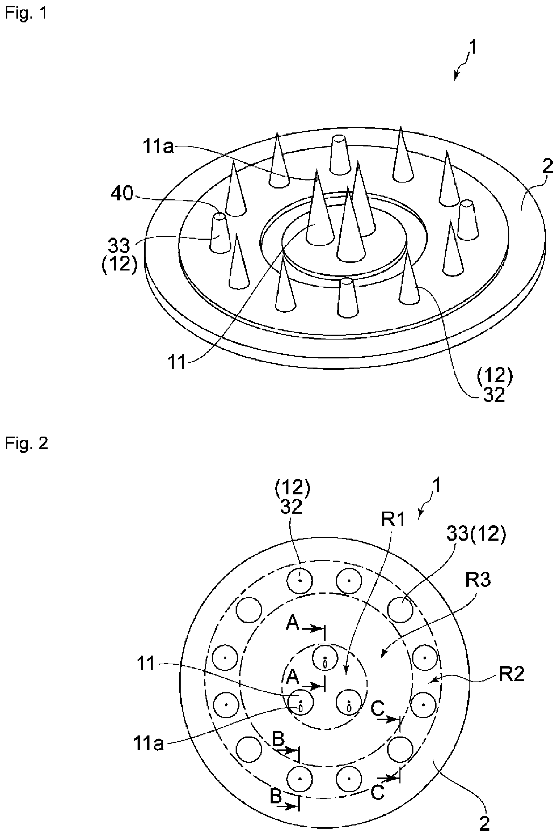

The projections of the injection needle 1 preferably include a first projection 11 having a conical shape and including an opening 11a in a distal end portion thereof and a second projection 12 not including an opening in a distal end portion thereof. An example of this is illustrated in Fig. 1. In the example illustrated in Fig. 1, the first projection 11 and the second projection 12 are fine projections, and are preferably fine projections having a protruding height of 5 000 μm or less.

The conical shape of the first projection 11 is typically a substantially circular conical shape but may be a polygonal pyramid such as a quadrangular pyramid. The substantially circular conical shape encompasses not only a circular conical shape but also an eccentric circular conical shape, in which the center and the distal end of the projection are at different locations.

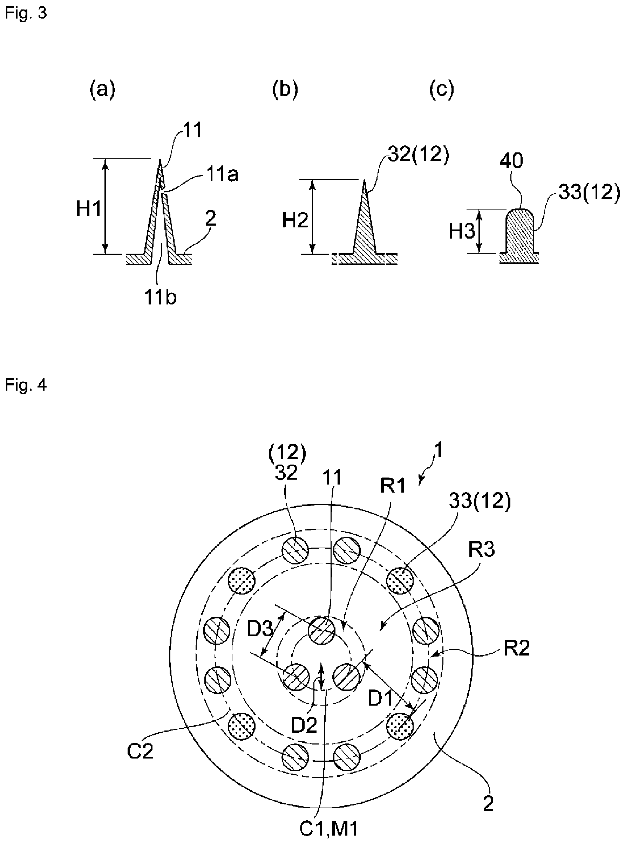

The first projection 11 is preferably a fine projection (i.e., so-called microneedle) including a hollow portion 11b (see Fig. 3(a)).

The second projection 12 may be hollow or may be solid. In the example illustrated in Fig. 1, the second projection 12 is solid (see Figs. 3(b) and (c)). A solid second projection 12 can reduce the amount of the liquid, such as a drug, remaining in the injection needle 1 when the liquid is injected from the injection needle 1.

As illustrated in Fig. 1, the second projections 12 preferably include a stimulation projection having a conical shape, 32 and a puncture depth-controlling projection (hereinafter also referred to as "control projection") having a columnar shape, 33. The control projection 33 includes a distal end surface serving as a puncture depth-controlling portion 40.

It is preferred that the distal end surface of the control projection 33 should be flat, that is, have a linear shape extending in a horizontal direction, or should be a curved line protruding in the protruding direction of the control projection 33.

As with the first projection 11, the conical shape of the stimulation projection 32 may be a substantially circular conical shape or may be a polygonal pyramid. The columnar shape of the control projection 33 may be a cylindrical shape or a polygonal columnar shape such as a quadrangular columnar shape.

The injection needle 1 preferably includes, as the second projections 12, the stimulation projection 32 having a conical shape, or the control projection 33 having a columnar shape, and more preferably includes both.

The injection needle 1 preferably includes the first region including one or two or more first projections 11, R1 in plan view. The first region R1 is preferably located in the central region of the sheet base portion 2 in plan view. The central region is, for example, a circular region having a diameter of one-third, preferably one-half, of the diameter of the sheet base portion 2. In the illustrated example, the center of the sheet base portion 2 and the center of the first region R1 coincide with each other, but the centers may deviate from each other.

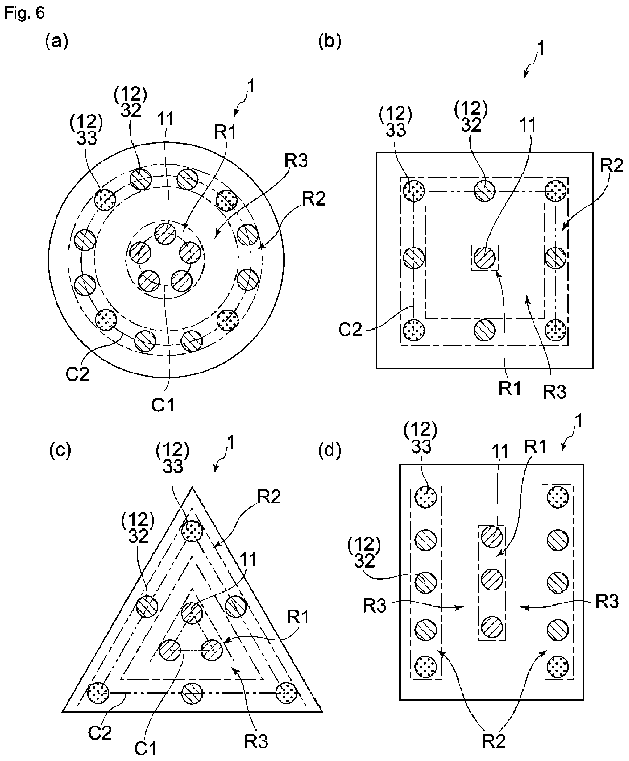

The number of the first projections 11 included in the first region R1 may be one or more. Preferably, multiple first projections 11 are arrayed and collectively form a given shape as a whole. The multiple first projections 11 are preferably arrayed in, for example, a triangular shape (see Fig. 2), a circular shape (see Fig. 6(a)), or a linear shape (see Fig. 6(d)).

The multiple first projections 11 are preferably arranged such that an imaginary line connecting the centers of the first projections 11, C1 forms an annular shape in plan view (see Fig. 2, Fig. 6(a), and Fig. 6(c)). Here, "annular" means a closed shape and encompasses not only a circular shape but also a polygonal shape, for example. In a case where the first projections 11 are arranged such that the imaginary line C1 forms an annular shape, preferably no first projection 11 is disposed entirely within the region enclosed by the imaginary line C1.

In the injection needle 1 illustrated in Fig. 2, the centers of the multiple first projections 11 are located on the same circle.

The injection needle 1 preferably includes the second region including two or more of the second projections 12, R2. It is preferable that the second region R2 surrounds or sandwiches the first region R1. In the example illustrated in Fig. 2, the second region R2 surrounds the first region R1.

The second projections 12 included in the second region R2 are preferably arranged such that an imaginary line connecting centers of the second projections 12 forms an annular shape in plan view. For example, for the second projections 12, the imaginary line connecting the centers of the second projections 12, C2 may have a circular annular shape (see Fig. 4 and Fig. 6(a)), may have a quadrangular ring shape (see Fig. 6(b)), or may have a triangular annular shape (see Fig. 6(c)). In the injection needle 1 illustrated in Fig. 2, the centers of the multiple second projections 12 are located on the same circle.

Fig. 6(d) illustrates an example in which the second regions R2 sandwich the first region R1. In a case where the second regions R2 sandwich the first region R1, the first region R1 preferably does not extend from the second regions R2.

Next, the first region R1 and the second region R2 will be described in detail.

As illustrated in Fig. 2 and Fig. 6(a), when multiple first projections 11 are collectively present in a central region of the sheet base portion 2 in plan view, the region inside the smallest circle surrounding the multiple first projections 11 is the first region R1.

As illustrated in Fig. 2 and Fig. 6(a), when multiple second projections 12 are disposed so as to surround the first region R1, the region between the smallest circle surrounding the multiple second projections 12 and the largest circle inscribed in the multiple second projections 12 is the second region R2.

When a single first projection 11, instead of the multiple first projections 11, is present in the central region of the sheet base portion 2 in plan view, the region inside the smallest circle surrounding the first projection 11 is the first region R1.

When multiple second projections 12 are arranged in a polygonal shape so as to surround the multiple first projections 11 collectively present in the central region or the single first projection 11 present in the central region, the region inside the smallest shape similar to the polygonal shape, the smallest shape surrounding the multiple first projections 11, may be defined as the first region R1 (see Figs. 6(b) and (c)).

When multiple second projections 12 are arranged in a polygonal shape, the region between the smallest line having a shape similar to the polygonal shape and surrounding the multiple second projections 12 and the largest line having a shape similar to the polygonal shape and inscribed in the multiple second projections 12 in plan view may be defined as the second region R2 (see Fig. 6(b) and (c)).

When multiple first projections 11 are arranged side by side in one direction, the region inside the smallest rectangle surrounding the multiple first projections 11 is the first region R1 (see Fig. 6(d)).

When multiple second projections 12 are arranged so as to sandwich the first region R1 (see Fig. 6(d)), the region inside the smallest rectangle surrounding the second projections 12 disposed on one side of the first region R1 and the region inside the smallest rectangle surrounding the second projections 12 disposed on the other side of the first region R1 are each the second region R2.

The injection needle 1 includes the opening 11a in the first projection 11, and thus serves as a liquid injection tool that can be used for intradermal administration of a liquid such as a drug.

Typically, the opening 11a is formed at the distal end portion of the first projection 11, and the hollow portion 11b of the first projection 11 communicates with the outside through the opening 11a. The opening 11a is a hole penetrating, in the thickness direction, the base material sheet forming the first projection 11, and is preferably located at the distal end portion of the first projection 11 having a conical shape. The hollow portion 11b of the first projection 11 functions as a storage portion or a passage for the liquid ejected from the opening 11a to the outside. Here, the distal end portion of the first projection 11 means the region on the distal end side relative to the intermediate location in the protruding height H1, which is from the surface of the sheet base portion 2 to the distal end of the first projection 11. Fig. 3(a) illustrates this specific example.

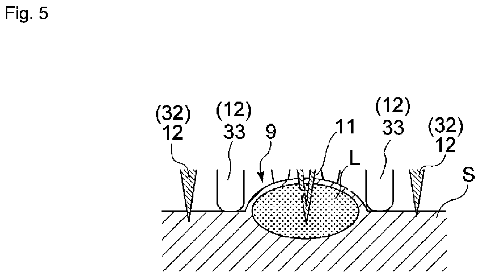

When the injection needle 1 is used for intradermal administration of a drug, the distal end portion of the first projection 11 is inserted into the skin S, and a drug L in liquid form stored in the hollow portion 11b of the first projection 11 is injected into the skin S from the opening 11a of the distal end portion (see Fig. 5). The hollow portion 11b of the first projection 11 thus functions as the storage portion of the drug typically when the drug is not supplied from the outside.

In a case where the drug is supplied from the outside, for example, in a case where a drug feeder (not illustrated) such as a syringe is used together with the injection needle 1, the hollow portion 11b functions as a passage of the drug. The drug feeder may be configured such that a sheet-like member is disposed on the back side of the injection needle 1 to form a drug accommodation space between the back side of the injection needle 1 and the sheet-like member, and in such a case, the drug is fed to the first projection 11 by pressing the sheet-like member (refer to paragraph [0011] of JP 2020-096790 A).

In the injection needle 1, the shortest distance among distances between the first projection 11 and the second projections 12 is designated as a distance D1 (see Fig. 4), and the distance D1 is preferably from 2.0 mm to 10 mm. The distance D1 within the range described above brings the following advantages.

The distal end portion of the first projection 11 is inserted into the skin S and the drug L is injected into the skin S; on this occasion, the skin rises by the injected drug L and a wheal 9 is formed (see Fig. 5). At this time, it is possible to prevent the second projection 12 from suppressing the rising of the skin, or that is, the skin readily rises, when the distance D1 is within the range described above. Thus, for example, even when an injection volume is large, a liquid such as the drug L can readily enter the body, and the liquid can be efficiently injected without leakage. The inventors examined a relationship between the liquid volume V injected into the skin S and the radius R of the formed wheal by the method below.

Relationship Between Liquid Volume and Radius of Wheal

Excised skin of a Göttingen mini pig (male, 6-week-old, ventral part; Oriental Yeast Co., Ltd.) stored at -20°C was transferred to a refrigerator (4°C) the day before the test and thawed. The excised skin was placed on 5 to 7 sheets of Kim Towels, brought to room temperature, shaved with a shaver, and wiped across the surface thereof with an alcohol swab before administration.

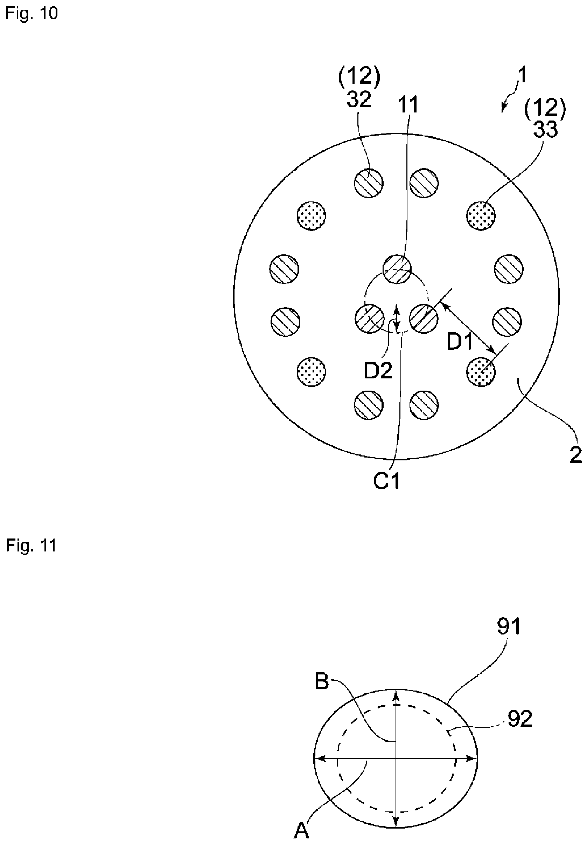

Powdered rhodamine B (Sigma-Aldrich) was added to ultrapure water to 0.5 mg/mL, and a microsyringe (Agilent) was filled with the resultant. The microsyringe and the injection needle were coupled to form an injection tool. Then, the injection tool was attached to a puncture device, and air was removed. The liquid volume in the microsyringe was set to 10, 25, 50, 100, or 250 μL, and excess liquid on the injection needle was absorbed and removed by a Kim Wipe. The arrangement of the projections of the injection needle is illustrated in Fig. 10. The height of the first projection 11, H1 of the injection needle was 2.0 mm, the height of the stimulation projection 32, H2 was 1.4 mm, and the height of the control projection 33, H3 was 0.9 mm. The distance D1, which was the shortest distance among the distances between the first projection 31 and the second projections 12, was 2.2 mm. The radius D2, which was the radius of the imaginary circle formed by the imaginary line connecting the centers of the first projections 11, C1, was 0.75 mm.

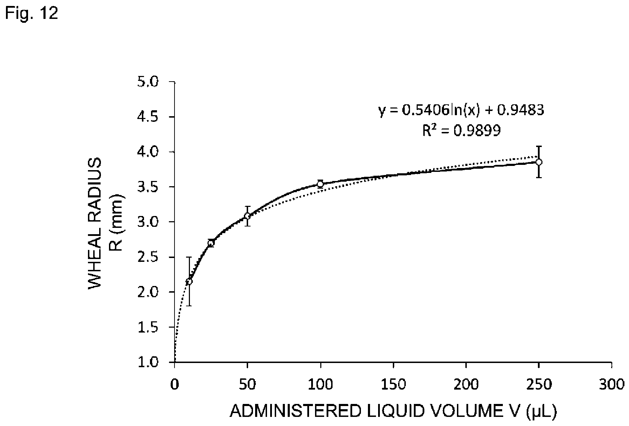

The skin was perpendicularly punctured with the injection needle using the puncture device, and an appropriate amount of the rhodamine B aqueous solution was intradermally administered. After the administration, the injection needle was pulled out, and the outline of the wheal (region that appears whiter and raised more than the surroundings) was immediately marked with a magic marker. The length of the major axis, A and the length of the minor axis, B of the wheal were measured using a vernier caliper. Fig. 11 illustrates a plan view schematically illustrating the wheal. In Fig. 11, reference sign 91 denotes the outline of the wheal, and reference sign 92 denotes the liquid in the wheal. Administration was made in triplicate for each dose. The average value of the length of the major axis, A and the length of the minor axis, B was divided by 2, and the value obtained was defined as the radius R of the wheal. A logarithmic approximation curve was calculated using the spreadsheet software "Excel" available from Microsoft Corporation, and the results are shown in Fig. 12. Details of the puncture device will be described later.

In the injection needle 1, a certain amount of interval is preferably provided between the first projection 11 and the second projection 12 to less cause suppression of the rising of the skin by the second projection 12. The certain amount of interval provided between the first projection 11 and the second projection 12 secures room for the skin to rise around the first projection 11, more specifically, between the first projection 11 and the second projection 12, when the drug L is injected into the skin S using the injection needle 1. Then, the rising of the skin is less likely to be suppressed by the second projection 12, whereby the liquid can be efficiently injected.

In view of ensuring the certain amount of interval between the first projection 11 and the second projection 12, the distance D1 is preferably 2.0 mm or greater.

When a drug is injected into the skin S by the injection needle 1, a wheal is rapidly formed even with a small injection volume, as illustrated in Fig. 12, for example. In this occasion, when the distance D1 is 2.0 mm or greater, the second projection 12 is less likely to inhibit the formation of the wheal 9 in the initial period, whereby the injectability of the liquid can be improved.

In view of avoiding impairment of the injectability of the liquid caused by inhibiting the rising of the wheal 9, the distance D1 is preferably one-half or greater, more preferably two-thirds or greater, and even more preferably greater, than the radius R of the wheal 9 formed when the drug L is injected into the skin S by the injection needle 1. The wheal radius R herein can be calculated by equation (1) below. In equation (1), V is a liquid volume injected into the skin S by the injection needle 1. The liquid to be injected is the liquid used in the "Relationship Between Liquid Volume and Radius of Wheal" described above. In equation (1), ln represents a natural logarithm.

$$R = 0.5406 \ln(V) + 0.9483 \qquad (1)$$

The reason why the ratio of the distance D1 to the wheal radius R calculated by equation (1) below is preferably greater than or equal to the ratios described above, and why the distance D1 is particularly preferably greater than the wheal radius R, is now described. For example, when the drug L is injected into the skin S using the injection needle 1 including only a single first projection 11, the first projection 11 inserted into the skin S is located at a center of the wheal 9. Accordingly, when the distance D1 is larger than the wheal radius R of the wheal 9, the second projection 12 is less likely to inhibit wheal formation during drug injection, making it possible to effectively prevent the second projection 12 from suppressing injection of the drug.

When the drug L is injected into the skin S by using the injection needle 1 including multiple of the first projections 11, a wheal is formed around each first projection 11 inserted into the skin S. Accordingly, when the distance D1 for each first projection 11 is larger than the wheal radius R of the wheal formed by each first projection 11, the second projection 12 is less likely to inhibit wheal formation during drug injection, making it possible to effectively prevent suppressing of injection of the drug.

The distance D1 is preferably 2.05 mm or greater, more preferably 2.1 mm or greater, in view of more remarkably exhibiting the effect of effectively preventing the second projection 12 from suppressing the rising of the skin. More specifically, when the liquid volume V injected into the skin S by the injection needle 1 is less than 10 μL, the distance D1 is preferably 2.0 mm or greater, more preferably 2.1 mm or greater. When the liquid volume V is 10 μL or greater, the distance D1 is preferably 2.1 mm or greater, more preferably 2.2 mm or greater.

The distance D1 is preferably 10 mm or less, more preferably 7 mm or less, and even more preferably 4 mm or less, in view of improving handleability by reducing the size of the injection needle 1 and effectively suppressing drawing of the skin by the stimulation projection 32.

The stimulation projection 32 can enhance an immunity-inducing effect when it is inserted into the skin. In view of significantly exhibiting this effect, the injection point of the injection needle 1, that is, the site where the first projection 11 is inserted is preferably close to the site where the stimulation projection 32 is inserted. Specifically, the distance D1 is preferably at most the upper limit of the range described above.

Here, the distance between the first projection 11 and the second projection 12 means the distance between the apex of the first projection 11 and the apex of the second projection 12. When the second projection 12 is the control projection 33, the distance between the first projection 11 and the second projection 12 means the distance between the apex of the first projection 11 and the center of the control projection 33 in plan view.

When the first region R1 includes multiple of the first projections 11 as in the example illustrated in Fig. 1, the distance D1 is preferably within the range described above for each of the multiple of the first projections 11.

The injection needle 1 preferably includes, between the first region R1 and the second region R2, a projection-free region R3, which does not include the first projection 11 or the second projection 12. Specific examples of such a case are illustrated in Fig. 4 and Figs. 6(a) to (d).

The projection-free region R3 is a region in which the distance D1 is within the range described above for each of the first projections 11 included in the first region R1. The projection-free region R3 is a region having a certain amount of width and, in the example illustrated in Fig. 4, the width of the projection-free region R3 is the same as the distance D1. The projection-free region R3 is preferably a region that includes neither at least the distal ends of the first projection 11 nor the second projection. The first projection 11 and the second projection 12 may rise sharply or rise gently from the sheet base portion 2. However, when the first projection 11 or the second projection 12 rises gently from the sheet base portion 2, for example, the projection-free region R3 may include the base portion of the first projection 11 or the second projection 12.

When the first projection 11 or the second projection 12 rises gently from the sheet base portion 2, the distance between the first projection 11 and the second projection 12 at the level corresponding to 80% of the height H1 of the first projection 11 may be regarded as the width of the projection-free region R3. When the first projection 11 or the second projection 12 rises sharply from the sheet base portion 2, the distance between the base portions of the first projection 11 and the second projection 12 may be regarded as the width of the projection-free region R3.

The projection-free region R3 preferably surrounds the first region R1 in plan view of the sheet base portion 2. Preferably, the projection-free region R3 has a certain amount of width and continuously surrounds the periphery of the first region R1. Specific examples of such a case are illustrated in Fig. 4 and Figs. 6(a) to (c).

The projection-free regions R3 may sandwich the first region R1 in plan view of the sheet base portion 2. A specific example of such a case is illustrated in Fig. 6(d).

The injection needle 1 including the projection-free region R3 allows the skin to rise without restriction when the liquid is injected into the skin by the injection needle 1, whereby the liquid can be more efficiently injected.

The plan-view shape of the projection-free region R3 is not particularly limited. However, because a wheal occurs in a circular shape, the plan-view shape is preferably an annular shape, and more preferably a circular annular shape. For example, the shape may be a circular ring shape (see Fig. 4 and Fig. 6(a)), a rectangular ring shape (see Fig. 6(b)), a triangular ring shape (see Fig. 6(c)), or the like.

In the injection needle 1, the second projections 12 preferably include the stimulation projection 32 and the control projection 33. In a case where the second projections 12 include the stimulation projection 32, the stimulation projection 32 is also inserted into the skin when the injection needle 1 is pressed against the skin to insert the distal end side of the first projection 11 into the skin. By inserting the stimulation projection 32 into the skin, it is possible to prevent the drawing of the skin to thereby improve puncturability. Furthermore, when the stimulation projection 32 is inserted into the skin, the stimulation projection 32 stimulates the skin to exert the promoting effect on the blood flow, thereby enhancing the immunity-inducing effect. Thus, the injection needle 1 in this configuration can readily inject the liquid into the skin by the first projection 11 and also can promote the blood flow by the stimulation projection 32, thereby enhancing the immunity-inducing effect.

The presence or absence of a blood flow-promoting effect can be determined by various known methods and, for example, by determining whether a flare reaction occurs in the skin or by visualizing the blood flow distribution with a laser blood flow meter.

In a case where the second projections 12 include the control projection 33, the puncture depth-controlling portion 40 of the control projection 33 comes into contact with the surface of the skin when the respective distal end sides of the first projection 11 and the stimulation projection 32 are inserted into the skin. This stops the insertion of the first projection 11 and the stimulation projection 32 and prevents the first projection 11 and the stimulation projection 32 from being inserted into a deeper area of the skin. In other words, the puncture depth-controlling portion 40 functions as a stopper that limits the insertion depth of the first projection 11 and the stimulation projection 32.

Thus, the injection needle 1 can be configured such that the insertion depth of the first projection 11 and the stimulation projection 32 into the skin can be controlled, whereby the drug is intradermally administered by the injection needle 1 to a desired depth in the skin while effectively promoting the blood flow. Therefore, by adjusting the insertion depth of the first projection 11 according to the type of the drug or other conditions, it is possible to maximize the effect of the drug. Furthermore, by adjusting the insertion depth of the stimulation projection 32 according to the desired degree of the blood flow-promoting effect, it is possible to effectively produce the blood flow-promoting effect.

In the injection needle 1, the first projection 11 is preferably higher than the second projection 12. This results in that the second projection 12 comes into contact with the skin after the first projection 11 is inserted into the skin. This makes it possible to efficiently insert the first projection into the skin and also to prevent the second projection 12 from drawing the skin. Subsequently, the first projection 11 can smoothly puncture the skin to a desired depth. While the second projections 12 typically include the stimulation projection 32 and the control projection 33, the first projection 11 is preferably higher than each of the stimulation projection 32 and the control projection 33.

In view of significantly reducing pain associated with intradermal administration or reliably injecting the drug from the first projection 11 into the skin S, the difference between the protruding height of the first projection 11, H1 and the protruding height of the control projection 33, H3 (i.e., the difference H1 - H3) is preferably 100 $\mu$m or greater, more preferably 300 $\mu$m or greater.

In view of not damaging the skin more than necessary, the difference H1 - H3 is preferably 4 995 $\mu$m or less, more preferably 3 000 $\mu$m or less.

In view of significantly reducing the pain associated with the blood flow-promoting effect of the stimulation projection 32 and sufficiently inserting the stimulation projection into the skin having elasticity, preferably the protruding height of the stimulation projection 32, H2 is equal to or higher than the protruding height of the control projection 33, H3, or that is, H2 ≥ H3, and preferably H2 > H3.

From the same viewpoint, the difference between the protruding height of the stimulation projection 32, H2 and the protruding height of the control projection 33, H3 (i.e., the difference H2 - H3) is preferably 0 µm or greater, more preferably 100 µm or greater, and even more preferably 400 µm or greater.

In view of not damaging the skin more than necessary, the difference H2 - H3 is preferably 4 995 µm or less, more preferably 3 000 µm or less.

In view of more reliably exhibiting the blood flow-promoting effect and ensuring reliable puncture by the first projection 11 to achieve efficient injection, the first projection 11 preferably is higher than the stimulation projection 32. More specifically, the difference between the protruding height of the first projection 11, H1 and the protruding height of the stimulation projection 32, H2 (i.e., the difference H1 - H2) is preferably 10 µm or greater, more preferably 100 µm or greater.

In view of readily obtaining both ease of liquid injection and the blood flow-promoting effect, the difference H1 - H2, is preferably 4 995 µm or less, more preferably 3 000 µm or less.

The protruding height of the first projection 11, H1 is preferably 105 µm or greater, more preferably 150 µm or greater. When the first projection 11 having such a protruding height H1 is pressed against the skin, the first projection 11 is successfully inserted into the skin to exhibit improved puncturability and also to reliably inject the drug into the skin S, even if the skin deforms.

In view of low invasiveness, the protruding height of the first projection 11, H1 is preferably 5 000 µm or less, more preferably 4 000 µm or less, and even more preferably 3 000 µm or less.

In view of stimulating the skin and promoting blood flow by pressing the stimulation projection 32 against the skin, the protruding height of the stimulation projection 32, H2 is preferably 5 µm or higher, more preferably 20 µm or higher.

In view of low invasiveness, the protruding height of the stimulation projection 32, H2 is preferably 5 000 µm or less, more preferably 4 000 µm or less, and even more preferably 3 000 µm or less.

In view of minimizing skin invasion and improving puncturability by maintaining the distance between the base material and the skin, the protruding height of the control projection 33, H3 is preferably 5 µm or higher, more preferably 10 µm or higher.

In view of improving the puncturability of the first projection 11, the protruding height of the control projection, H3 is preferably 4 000 µm or less, more preferably 3 000 µm or less, and even more preferably 2 000 µm or less.

In the injection needle 1 illustrated in Fig. 1, an imaginary circle M1 (see Fig. 4) is provided, which is the smallest imaginary circle including the centers of all first projections 11 included in the first region R1; at this occasion, the radius of the imaginary circle M1 (i.e., the radius D2; see Fig. 4) is preferably smaller than the distance D1 (see Fig. 4), which is the shortest distance among the distances between the first projection 11 and the second projections 12. In a case where the center of the first projection 11 is located on a circumference of the imaginary circle M1, the center of the first projection 11 is considered to be included in the imaginary circle M1. In a case where the radius D and the distance D1 satisfy the relationship as described above, formation of the wheal 9 is less likely to be inhibited when the liquid is injected into the skin by the injection needle 1, whereby the liquid can be injected more efficiently. In view of more remarkably exhibiting this effect, the ratio of the radius D2 to the distance D1, D1/D2, is preferably 1.0 or greater, more preferably 1.5 or greater.

In view of dispersing the liquid injection site relative to the wheal to be formed and reducing the size of the injection needle 1, the ratio D1/D2 is preferably 20 or less, more preferably 10 or less.

In the injection needle 1, the distance D3 (see Fig. 4), which is the shortest distance among distances between the first projections 11 in the first region R1, is preferably shorter than the distance D1. Owing to this, the formation of wheal 9 is less inhibited when the liquid is injected into the skin by the injection needle 1, whereby the liquid can be more efficiently injected. In view of more remarkably exhibiting this effect, the ratio of the distance D3 to the distance D1, D1/D3, is preferably 1.0 or greater, more preferably 1.5 or greater.

In view of dispersing the liquid injection site relative to the wheal to be formed and reducing the size of the injection needle 1, the ratio D1/D3 is preferably 20 or less, more preferably 10 or less.

In the injection needle 1, in view of a balance between ease of puncture plus liquid injection and the blood flow-promoting effect, the ratio of the number of the second projections 12 to the number of the first projections 11 (the number of the second projections 12/ the number of the first projections 11) is preferably 2.4 or greater, more preferably 3.0 or greater, and even more preferably 3.5 or greater.

In view of low invasiveness, the ratio is preferably 10 or less, more preferably 8 or less, and even more preferably 6 or less.

In the injection needle 1, in view of enabling efficient injection of the liquid by the injection needle 1, the first region R1 preferably includes two or more first projections 11, more preferably three or more first projections 11.

In view of facilitating favorable injection of the liquid from all first projections 11, the number of first projections 11 is preferably 20 or less, more preferably 15 or less, and even more preferably 10 or less.

In the injection needle 1, the second region R2 may include the first projection 11, but preferably does not include the first projection 11 (see Fig. 4 and Fig. 6). In a case where the second region R2 does not include the first projection 11, only the first region R1 of the injection needle 1 corresponds to a region where the liquid can be injected, making it possible to limit the locations of wheals to the first region and prevent inhibition of the formation of wheals at other locations.

The injection needle 1 may include, as the second projection 12, a composite second projection 52 in which the puncture depth-controlling portion 40 is integrally formed around the stimulation projection 32. An example thereof is illustrated in Fig. 7. In the example illustrated in Fig. 7, the composite second projection 52 is formed by integrally forming the stimulation projection 32 with the puncture depth-controlling portion 40 on the base portion side of the stimulation projection 32 and around the outer peripheral portion of the stimulation projection 32. More specifically, an enlarged diameter portion 41 is formed on the base portion side, and a stepped portion projecting outward in the radial direction of the stimulation projection 32 is formed in the upper end portion of the enlarged diameter portion 41. The stepped portion serves as the puncture depth-controlling portion 40.

When the first projection 11 and the composite second projection 52 on the distal end side are inserted into the skin, the puncture depth-controlling portion 40 comes into contact with the surface of the skin to thereby stop the insertion of the first projection 11 and the composite second projection 52, and the first projection 11 and the composite second projection 52 are thus prevented from being inserted into a deeper area of the skin. Since the puncture depth-controlling portion 40 is integrally formed around the stimulation projection 32 in the composite second projection 52, it is possible to more reliably prevent the composite second projection 52 from being inserted into a deeper area of the skin.

Next, constituent materials of the injection needle 1 will be described.

In view of ensuring a handleability of the materials, and a strength and fabricability of the injection needle, and in view of ensuring the hardness of the first projection 11 and the second projection 12 to facilitate the injection of the liquid and also to improve the blood flow-promoting effect, the injection needle 1 preferably contains a thermoplastic resin. The injection needle is more preferably formed of a base material sheet containing a thermoplastic resin.

The thermoplastic resin may include one or two or more selected from polyolefin, polyester, polyamide, polyamide-imide, polyether ether ketone, polyetherimide, polyvinyl chloride, acrylic resin, and polystyrene resin.

The polyolefin may include one or two or more selected from polypropylene, polyethylene, and others.

The polyester may include one or two or more selected from polyethylene terephthalate, polyfatty acid ester, polylactic acid, polycaprolactone, polybutylene succinate, and others.

The polyamide may include one or two or more selected from nylon and others.

In view of biodegradability, preferably a polyfatty acid ester is included.

Specifically, one or two or more selected from polylactic acid and polyglycolic acid may be included as the polyfatty acid ester.

The proportion of the mass of the thermoplastic resin contained in the injection needle 1 in the total mass of the injection needle 1 is preferably 50% or greater, more preferably 70% or greater, and even more preferably 90% or greater, in view of improving the fabricability and the dimensional stability of the injection needle.

The proportion of the mass of thermoplastic resin in the total mass the injection needle is preferably 100% or less, more preferably 98% or less, and even more preferably 96% or less, in view of incorporating, for example, a functional agent into the injection needle to provide various effects.

Here, examples of the various functional agents that can be used include an antibacterial agent, a bactericide, a humectant, a flow improver, an antistatic agent, and a colorant.

The drug to be injected into the skin by the injection needle 1 can be selected as appropriate according to the application of the injection needle 1.

The injection needle 1 can be used not only for the intradermal administration of drugs but also for subcutaneous administration, for example. The drug to be injected into the skin by the injection needle 1 may be a drug for intradermal administration or may be a drug for subcutaneous administration. The drug for intradermal administration means a drug for which intradermal administration is recommended. The drug for subcutaneous administration means a drug for which subcutaneous administration is recommended. The injection needle 1 facilitates intradermal administration of the drug, and thus the drug injected into the skin by the injection needle 1 is preferably a drug for intradermal administration, more preferably a vaccine drug for intradermal administration. In particular, when a vaccine drug for intradermal administration is used, it is possible to reliably intradermally administer the drug and further efficiently enhance antigen recognition by the immune system as compared with subcutaneous administration, and thus enhancement of the effect of the vaccine can be expected. The skin includes the epidermis, the dermis, and the subcutaneous tissue, in this order from the body surface side, and a relatively large number of immune cells are present in the dermis. Therefore, when a vaccine drug for intradermal administration is intradermally administered by the injection needle 1, the drug is preferably administered into the dermis in view of enhancing the effect of the vaccine.

Examples of the drug include vaccines for preventing infectious diseases such as hepatitis A, hepatitis B, hepatitis C, influenza, COVID-19, tuberculosis, rabies, polio, chicken pox, rubella, measles, tetanus, shingles and the like; vaccines for treating chronic hepatitis B, tuberculosis, rabies, malignant neoplasms, shingles, and the like; analgesics for patients with cancer; insulin; biological preparations; gene therapeutic agents; injectable preparations; and dermatologic preparations, and the drug may include one or two or more selected from these.

The first projection 11 of the injection needle 1 punctures the skin, and thus the injection needle 1 can be applied not only to pharmacologically active substances for transdermal administration in the related art, but also to pharmacologically active substances requiring subcutaneous injection.

The skin S into which the drug L is injected by the injection needle 1 may be the human skin or may be the skin of an animal other than a human.

The present invention includes an injection tool including an injection needle and a drug feeder to be filled with a drug and coupled to the injection needle. As the injection needle included in the injection tool of the present invention, the injection needle 1 of the present invention described above can be used. For example, a syringe, a tube, or an electric injector can be used as the drug feeder. The drug feeder may include a drug storage portion pre-filled with a drug.

When the injection tool of the present invention is used to inject the drug, the formation of the wheal 9 is less likely to be inhibited, whereby the drug can be efficiently injected. Furthermore, the injection tool of the present invention allows for reliable intradermal administration of the drug, making it possible to further improve the effect of a drug for intradermal administration by using a drug feeder filled with the drug for intradermal administration, as the drug feeder.

The present invention includes a kit including an injection needle, a drug feeder to be filled with a drug, and the drug. As the injection needle included in the kit of the present invention, the injection needle 1 of the present invention described above can be used. As the drug feeder, any of those listed above as the drug feeder for the injection tool of the present invention can be used. As the drug, any of those listed above as the drug used with the injection needle 1 of the present invention can be used. The kit may include a puncture device having a structure by which a speed can be applied to the injection tool in a direction perpendicular to the administration site. The puncture device is a tool used for puncturing the skin S with an injection needle, and includes, for example, a spring as the structure for applying a speed for puncturing the skin with the injection needle. The term "speed" means a speed at which the first projection 11 of the injection needle 1 punctures the skin.

When the kit of the present invention is used to inject the drug, formation of the wheal 9 is less likely to be inhibited, whereby the drug can be efficiently injected.

For example, the puncture device includes: a holding portion that holds the injection needle 1; a guide mechanism that regulates the moving direction of the injection needle 1 so that the first projection 11 of the injection needle 1 punctures the skin substantially perpendicularly; and a drive mechanism that moves the injection needle 1 along the guide mechanism. The drive mechanism preferably includes a driving means, such as a spring, that moves the injection needle 1 while controlling the puncture speed of the first projection 11 of the injection needle 1 at a predetermined speed. The drive mechanism is preferably configured such that the driving means can be manually operated.

The holding portion is, for example, a portion to which the injection needle 1 is attached, the portion being in an adapter member that can couple the injection needle 1 and the drug feeder to each other. The holding portion can be formed, for example, at one end portion of the adapter member. Preferably, the drug can be fed from the drug feeder to the first projection 11 of the injection needle 1 in a state in which the injection needle 1 and the drug feeder are coupled to each other via the adapter member. A means for attaching the injection needle 1 to the holding portion is not particularly limited and, for example, any desired means such as fitting, screwing, fusion bonding, and adhesion can be used. The holding portion and the injection needle 1 may respectively include engaging portions that engage with each other, and the injection needle 1 may be attached to the holding portion by engaging the engaging portions. Preferably, the other end portion of the adapter member is configured such that the drug feeder can be attached. For example, the other end portion of the adapter member is preferably configured such that a distal end opening of a syringe can be inserted thereinto.

The puncture device may have a body having a cylindrical shape, which is the main portion of the puncture device. The guide mechanism includes, for example, a guide hole provided in the side wall of the body of the puncture device, and an injection tool holder including a guide projection inserted into the guide hole. The opening of the guide hole has a shape extending in the axial direction of the body. The injection tool holder holds the injection tool formed by coupling the injection needle 1 and the drug feeder via the adapter member. The guide projection of the injection tool holder is inserted into the guide hole, whereby the moving direction of the injection tool holder is restricted to the axial direction of the body. The injection tool holder is disposed within the body.

The driving means of the drive mechanism is preferably configured to move the injection tool holder. When the driving means is a spring, the puncture device preferably includes a stopper mechanism for keeping the spring in a contracted state. The stopper mechanism is preferably such that the spring is kept in a contracted state by fixing the position of the guide projection.

A method for injecting a drug into the skin using the puncture device is now described.

First, a syringe serving as the drug feeder is filled with a drug. Specifically, in a state in which the distal end opening of the syringe is immersed in the drug, the plunger inserted into the syringe is pulled up, to suction the drug into the syringe. The injection needle 1 is attached to the syringe filled with the drug via the adapter member, to form the injection tool. The plunger of the syringe is pressed down to fill the first projection 11 of the injection needle 1 with the drug. The injection tool is held by the injection tool holder, and the injection tool is attached to the puncture device. The guide projection of the injection tool holder is pressed upward along the guide hole to contract the spring of the drive mechanism energizing the injection tool holder. The guide projection is fixed by the stopper mechanism to keep the spring in the contracted state (hereinafter, this state is also referred to as a "standby state"). The first projection 11 of the injection needle 1 in the puncture device in the standby state is brought close to the skin where the drug is to be administered. At this time, preferably the axial direction of the body of the puncture device is perpendicular to the skin. Then, the fixed state of the guide projection is released. This urges the injection needle 1 toward the skin and causes the first projection 11 to puncture the skin. Subsequently, the plunger of the syringe is pressed to inject the drug into the skin.

Although the present invention has been described on the basis of the preferred embodiments thereof, the present invention is not limited to the embodiments described above and can be modified as appropriate.

For example, although the second projection 12 is solid in the example illustrated in Fig. 3, the second projection 12 may be hollow. Further, in the example illustrated in Fig. 7, the composite second projection 52 is solid; however, the composite second projection 52 may be hollow. In the injection needle 1, the projections other than the first projection 11 are preferably solid. In this way, the liquid to be ejected from the opening 11a of the first projection 11 can be prevented from being stored in the other projections, making it possible to efficiently inject the liquid.

In the example illustrated in Fig. 1, the injection needle 1 has a flat sheet-like shape as a whole, but the overall shape of the injection needle 1 is not limited thereto. The injection needle 1 may be formed integrally with the adapter member. For example, the first and second projections 11, 12 may protrude on one side of a plate-like member, and a tubular portion functioning as the adapter member may be provided at the peripheral edge portion on the other side by bonding with adhesion or the like or by integral molding. In this case, the plate-like portion is the sheet base portion 2.

Examples

Hereinafter, the present invention will be more specifically described with reference to examples, but the present invention is not limited to these examples.

Example 1

An injection needle in the same configuration and having the same projection arrangement as those of the injection needle 1 illustrated in Figs. 1 and 2 was manufactured. Table 1 shows dimensions of each projection of the injection needle, the distance D1, which was the shortest distance among the distances between the first projection and the second projections, the radius of the smallest imaginary circle including the centers of all first projections included in the first region (i.e., the radius D2), the distance D3, which was the shortest distance among the distances between the first projections in the first region, and the proportion of the mass of the thermoplastic resin in the total mass of the injection needle. In the injection needle of example 1, the plan-view shape of the projection-free region was a circular annular shape surrounding the first region. The injection needle was formed by subjecting a base material sheet made of 100% polylactic acid as a thermoplastic resin to a projection-forming process using a processing needle to which ultrasonic vibration was applied.

[Table 1]

| | | | Example 1 | Example 2 | Comparative Example 1 | Comparative Example 2 |
|---|---|---|---|---|---|---|
| First projection | | Protruding height H1 ($\mu$m) | 2000 | 2000 | 2000 | 2000 |
| | | Number (projections) | 3 | 3 | 3 | 3 |
| Second projection | Stimulation projection | Protruding height H2 ($\mu$m) | 1400 | 1400 | 1400 | - |
| | | Number (projections) | 8 | 6 | 4 | - |
| | Puncture depth-controlling projection | Protruding height H3 ($\mu$m) | 900 | 900 | 900 | - |
| | | Number (projections) | 4 | 4 | 4 | - |
| H1 - H2 ($\mu$m) | | | 600 | 600 | 600 | - |
| H1 - H3 ($\mu$m) | | | 1100 | 1100 | 1100 | - |

(continued)

| | | Example 1 | Example 2 | Comparative Example 1 | Comparative Example 2 |
|---|---|---|---|---|---|
| H2 - H3 ($\mu$m) | | 500 | 500 | 500 | - |
| D1 (mm) | | 2.2 | 2.2 | 0.5 | - |
| D2 (mm) | | 0.75 | 0.75 | 0.75 | 0.75 |
| D3 (mm) | | 1.30 | 0.75 | 1.30 | 1.30 |
| D1/D2 | | 2.9 | 2.9 | 0.7 | - |
| D1/D3 | | 1.7 | 2.9 | 0.4 | - |
| Number of second projections/Number of first projections | | 4.0 | 3.3 | 2.7 | - |
| Proportion (%) of mass of thermoplastic resin in total mass of injection needle | | 100 | 100 | 100 | 100 |
| Evaluation | Puncturability | Good | Good | Good | Poor |
| | Wheal formability, administrability | Good | Good | Poor | Poor |

Example 2

An injection needle in the same configuration as that of the injection needle 1A illustrated in Fig. 1 and having the same arrangement as that in Fig. 6(d) was manufactured. This example was the same as example 1 except that the variables were as shown in Table 1. In the injection needle of example 2, the plan-view shape of each projection-free region was rectangular, and the projection-free regions sandwiched the first region.

Comparative Example 1

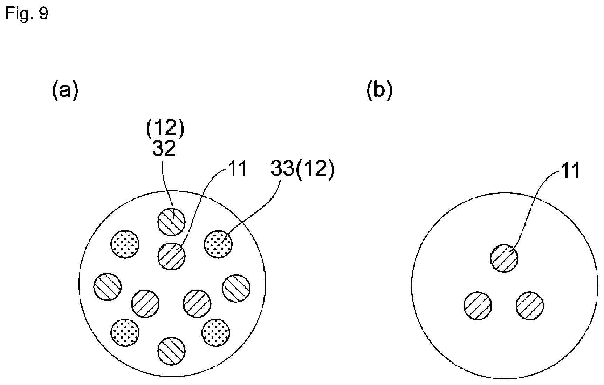

An injection needle including the first projection 11, the stimulation projection 32, and the control projection 33 was manufactured (see Fig. 9 (a)). This example was the same as example 1, except that the variables were as shown in Table 1 and that the projections 11, 32, 33 were arranged as illustrated in Fig. 9(a). The injection needle of comparative example 1 did not include a projection-free region.

Comparative Example 2

An injection needle including only the first projection 11 was manufactured (see Fig. 9(b)). The variables were as shown in Table 1. This example was the same as example 1 except that the stimulation projection 32 and the control projection 33 were not provided. The injection needle of comparative example 2 did not include the second region or the projection-free region.

Administration of Drug Solution

Using the injection needles of examples 1 and 2 and comparative examples 1 and 2, a drug solution was administered to the skin of a Göttingen mini pig by the method below.
Excised skin of a Göttingen mini pig (male, 6-week-old, ventral part; Oriental Yeast Co., Ltd.) stored at -20°C was transferred to a refrigerator (4°C) the day before the test and thawed. The excised skin was placed on 5 to 7 sheets of Kim Towels, shaved using a shaver, and wiped across the surface thereof with an alcohol swab before administration.
Powdered rhodamine B (Sigma-Aldrich) was added to ultrapure water to 0.5 mg/mL, and a microsyringe (Agilent) was filled with the resultant. The microsyringe was attached to the puncture device, any one of the injection needles of examples 1 and 2 and comparative examples 1 and 2 was attached thereto, and air was removed. The liquid volume in the microsyringe was set to 100 $\mu$L, and excess liquid on the injection needle was absorbed and removed by a Kim Wipe. The projection of the injection needle was caused to perpendicularly puncture the puncture target, and 100 $\mu$L of rhodamine B aqueous solution was injected.
After the drug solution was administered to the skin of the Göttingen mini pig by the method set forth in Administration of Drug Solution above, the puncturability and the administrability of each of the injection needles of examples 1 and 2 and comparative examples 1 and 2 were evaluated according to the evaluation criteria below. The results are shown in Table 1.

Evaluation Criteria of Puncturability

Good: Both (1) and (2) below are satisfied.

(1) When puncturing the skin, the first projection can be inserted to such a depth that liquid can be injected.
(2) When puncturing the skin, the first projection is not inserted too deeply and can be inserted to such a depth that a wheal is formed during liquid injection.

Poor: One or both of the above (1) and (2) are not satisfied.

Evaluation Criteria of Administrability

Good: Both (1) and (2) below are satisfied.

(1) Formation of a wheal can be visually confirmed immediately after administration of the drug solution, and wheal formation is not inhibited by the second projection.
(2) After administration, the surface of the administration site in the skin and the surface of the injection needle are wiped with a paper towel which has been weighed in advance, and the weight change of the paper towel before and after wiping is 5 mg or less.

Poor: One or both of the above (1) and (2) are not satisfied.
As shown in Table 1, the injection needles of examples 1 and 2 were graded "Good" for both puncturability and administrability, and it is understood that the first projection readily punctures the skin, and that the wheal formation is not readily inhibited during injection. As shown in Table 1, the injection needle of comparative example 1 was graded "Good" for puncturability but graded "Poor" for administrability. As shown in Table 1, the injection needle of comparative example 2 was graded "Poor" for both puncturability and administrability.
In particular, regarding puncturability of the injection needle of comparative example 2, when the skin was punctured with the first projection, the extracted skin was drawn, making it difficult to appropriately puncture the skin with the first projection, or the puncture was too deep, making it difficult to inject the drug solution to a predetermined depth. Further, regarding administrability as well, the drug solution leaked due to failure in the puncture of the skin with the first projection, or it was not possible to inject the drug solution to a predetermined depth due to too deep insertion of the first projection, whereby a wheal was not formed. It is thus clear that the injection needle of the present invention is less likely to suppress the rising of the skin when liquid is injected, and can thus efficiently inject the liquid.

Industrial Applicability

According to the present invention, the injection needle less causes suppression of the rising of the skin by the injected liquid to facilitate efficient injection of the liquid, when liquid is injected into the skin by the injection needle.

**Claims**

1. An injection needle comprising:

a first projection which is fine and protrudes from a sheet base portion, wherein the first projection has a conical shape, and includes an opening in a distal end portion thereof; and
a second projection which is fine and protrudes from the sheet base portion, wherein the second projection includes no opening in a distal end portion thereof; and
the injection needle including a first region and a second region in plan view; wherein the first region includes one or two or more of the first projections; the second region includes two or more of the second projections; and the second region surrounds or sandwiches the first region,
a shortest distance among distances between the first projection and the second projections, D1, is from 2.0 mm to 10 mm.

2. The injection needle according to claim 1, wherein

the first region includes two or more first projections, and
when a smallest imaginary circle including centers of all of the two or more first projections included in the first region is given, a radius of the smallest imaginary circle, D2, is smaller than the distance D1.

3. The injection needle according to claim 1 or 2, wherein

the first region includes two or more of the first projections, and
a shortest distance among distances between the two or more first projections in the first region, D3, is shorter than the distance D1.

4. The injection needle according to any one of claims 1 to 3, wherein
one or two or more of the first projections is higher than the two or more second projections.

5. The injection needle according to any one of claims 1 to 4, wherein

the two or more second projections include
a stimulation projection having a conical shape, and/or
a puncture depth-controlling projection including a distal end surface serving as a puncture depth-controlling portion.

6. The injection needle according to claim 5, wherein
the distal end surface of the puncture depth-controlling projection is flat or a curved line protruding in a protruding direction of the puncture depth-controlling projection.

7. The injection needle according to claim 5 or 6, wherein
the stimulation projection is higher than the puncture depth-controlling projection.

8. The injection needle according to any one of claims 5 to 7, wherein
the first projection is higher than each of the stimulation projection and the puncture depth-controlling projection.

9. The injection needle according to any one of claims 5 to 8, wherein
a difference $H1 - H3$, which is a difference between a protruding height of a first projection, $H1$, and a protruding height of the puncture depth-controlling projection, $H3$, is from 100 $\mu$m to 4995 $\mu$m, preferably from 300 $\mu$m to 3000 $\mu$m.

10. The injection needle according to any one of claims 5 to 9, wherein
a difference $H2 - H3$, which is a difference between a protruding height of a stimulation projection, $H2$ and a protruding height of the puncture depth-controlling projection, $H3$, is from 0 $\mu$m to 4 995 $\mu$m, more preferably from 100 $\mu$m to 3 000 $\mu$m, and even more preferably from 400 $\mu$m to 3 000 $\mu$m.

11. The injection needle according to any one of claims 5 to 10, wherein
a difference $H1 - H2$, which is a difference between a protruding height of a first projection, $H1$ and a protruding height of the stimulation projection, $H2$, is from 10 $\mu$m to 4 995 $\mu$m, preferably from 100 $\mu$m to 3 000 $\mu$m.

12. The injection needle according to any one of claims 5 to 11, wherein
a protruding height of a first projection, $H1$ is from 105 $\mu$m to 5 000 $\mu$m, preferably from 150 $\mu$m to 4 000 $\mu$m, more preferably from 150 $\mu$m to 3 000 $\mu$m.

13. The injection needle according to any one of claims 5 to 12, wherein
a protruding height of a stimulation projection, $H2$ is from 5 $\mu$m to 5 000 $\mu$m, preferably from 20 $\mu$m to 4 000 $\mu$m, more preferably from 150 $\mu$m to 3 000 $\mu$m.

14. The injection needle according to any one of claims 5 to 13, wherein
a protruding height of a puncture depth-controlling projection, $H3$ is from 5 $\mu$m to 4000 $\mu$m, preferably from 10 $\mu$m to 3000 $\mu$m, more preferably from 10 $\mu$m to 2000 $\mu$m.

15. The injection needle according to any one of claims 1 to 14, wherein

when a smallest imaginary circle including centers of all of the first projections included in the first region is given, a ratio D1/D2, which is a ratio of the distance D1 to a radius of the smallest imaginary circle, D2, is from 1.0 to 20, preferably from 1.5 to 10, and/or
a ratio D1/D3, which is a ratio of the distance D1 to a shortest distance among distances between the first projections in the first region, D3, is from 1.0 to 20, preferably from 1.5 to 10.

16. The injection needle according to any one of claims 1 to 15, wherein
when a smallest imaginary circle including centers of all of the first projections included in the first region is given, a ratio D1/D2, which is a ratio of the distance D1 to a radius of the smallest imaginary circle, D2, is from 1.0 to 20, preferably from 1.5 to 10.

17. The injection needle according to any one of claims 1 to 16, wherein
a ratio D1/D3, which is a ratio of the distance D1 to a shortest distance among distances between the first projections in the first region, D3, is from 1.0 to 20, preferably from 1.5 to 10.

18. The injection needle according to any one of claims 1 to 17, wherein
the first region includes from 2 to 20, preferably from 3 to 20, more preferably from 3 to 15 of the first projections.

19. The injection needle according to any one of claims 1 to 18, wherein
a ratio of a number of the second projections to a number of the first projections is from 2.4 to 10, preferably from 3.0 to 8, more preferably from 3.5 to 6.

20. The injection needle according to any one of claims 1 to 19, wherein
the second region does not include the first projection.

21. The injection needle according to any one of claims 1 to 20, comprising
a projection-free region that is present between the first region and the second region and does not include the first projection or the second projection.

22. The injection needle according to claim 21, wherein
in plan view of the sheet base portion, the projection-free region surrounds or sandwiches the first region.

23. The injection needle according to claim 21 or 22, wherein
the projection-free region has a certain amount of width and continuously surrounds a periphery of the first region.

24. The injection needle according to any one of claims 21 to 23, wherein
in plan view, the projection-free region has an annular shape, preferably a circular annular shape.

25. The injection needle according to any one of claims 1 to 24, wherein

the first region includes multiple of the first projections, and
the multiple first projections included in the first region are disposed such that an imaginary line connecting centers of the multiple first projections forms an annular shape in plan view.

26. The injection needle according to claim 25, wherein
the first projection is not disposed inside a region surrounded by the imaginary line.

27. The injection needle according to any one of claims 1 to 26, wherein
the second projections included in the second region are disposed such that an imaginary line connecting centers of the second projections forms an annular shape in plan view.

28. The injection needle according to any one of claims 1 to 27, wherein
the distance D1 is from 2.05 mm to 10 mm, preferably from 2.1 mm to 7.0 mm, more preferably from 2.1 mm to 4 mm.

29. The injection needle according to any one of claims 1 to 28, wherein
the injection needle includes a thermoplastic resin, and a proportion of a mass of the thermoplastic resin in a total mass of the injection needle is from 50% to 100%, more preferably from 70% to 98%, and even more preferably from 90% to 96%.

30. The injection needle according to any one of claims 1 to 29, wherein
the injection needle is used for intradermal administration of a drug.

31. An injection tool comprising:

the injection needle according to any one of claims 1 to 29; and
a drug feeder to be filled with a drug and coupled to the injection needle.

32. A kit comprising:

the injection needle according to any one of claims 1 to 29;
a drug feeder to be filled with a drug; and
the drug.

33. A kit comprising:

the injection needle according to any one of claims 1 to 29;
a drug feeder to be filled with a drug;
the drug; and
a puncture device configured to move the injection needle toward the skin to puncture the skin with the first
projection of the injection needle.

34. An injection tool comprising:

the injection needle according to any one of claims 1 to 29; and
a drug feeder to be filled with one or two or more drugs and coupled to the injection needle, wherein

the one or two or more drugs are selected from vaccines for preventing infectious diseases such as hepatitis A,
hepatitis B, hepatitis C, influenza, COVID-19, tuberculosis, rabies, polio, chicken pox, rubella, measles, tetanus,
shingles and the like; vaccines for treating chronic hepatitis B, tuberculosis, rabies, malignant neoplasms, shingles,
and the like; analgesics for patients with cancer; insulin; biological preparations; gene therapeutic agents; injectable
preparations; and dermatologic preparations.

35. A kit comprising:

the injection needle according to any one of claims 1 to 29;
a drug feeder to be filled with one or two or more drugs selected from vaccines for preventing infectious diseases
such as hepatitis A, hepatitis B, hepatitis C, influenza, COVID-19, tuberculosis, rabies, polio, chicken pox, rubella,
measles, tetanus, shingles and the like; vaccines for treating chronic hepatitis B, tuberculosis, rabies, malignant
neoplasms, shingles, and the like; analgesics for patients with cancer; insulin; biological preparations; gene
therapeutic agents; injectable preparations; and dermatologic preparations; and
the one or two or more drugs.

36. An injection tool comprising:

an injection needle; and
a drug feeder to be filled with an intradermal vaccine and coupled to the injection needle
the injection needle comprising:

a first projection which is fine and protrudes from a sheet base portion, wherein the first projection has a
conical shape and includes an opening in a distal end portion thereof; and
a second projection which is fine and protrude from the sheet base portion, wherein the second projection
includes no opening in a distal end portion thereof; and

the injection needle including a first region and a second region in plan view; wherein the first region includes one
or two or more first projections; the second region includes two or more second projections; and the second region
surrounds or sandwiches the first region,
wherein a shortest distance among distances between the first projection and the second projections, D1, is from 2.0 mm to 10 mm.

**37.** A method comprising:
puncturing the skin with the first projection of the injection needle according to any one of claims 1 to 29 using a puncture device comprising the injection needle and a drug feeder to be filled with a drug and coupled to the injection needle, wherein the puncture device is configured to move the injection needle toward the skin to puncture the skin with the first projection of the injection needle.

**38.** A method for administering a drug into the skin using a kit comprising the injection needle according to any one of claims 1 to 29, a drug feeder to be filled with a drug, and the drug,
the method comprising:

moving the injection needle toward the skin using a puncture device to puncture the skin with the first projection of the injection needle; and
administering the drug.

Fig. 1

Fig. 2

Fig. 3

(a)  (b)  (c)

Fig. 4

Fig. 5

Fig. 6

Fig. 7

Fig. 8

(a)

(b)

Fig. 9

(a)

(12)
32

11    33(12)

(b)

11

Fig. 10

Fig. 11

Fig. 12

## INTERNATIONAL SEARCH REPORT

| International application No. |
| --- |
| **PCT/JP2023/043025** |

| A. | CLASSIFICATION OF SUBJECT MATTER |
| --- | --- |

*A61M 37/00*(2006.01)i
FI:   A61M37/00 514; A61M37/00 500

According to International Patent Classification (IPC) or to both national classification and IPC

| B. | FIELDS SEARCHED |
| --- | --- |

Minimum documentation searched (classification system followed by classification symbols)

A61M37/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | JP 2017-38781 A (KAO CORPORATION) 23 February 2017 (2017-02-23) paragraphs [0011]-[0030], fig. 1-8 | 1, 3-4, 15, 17-30 |
| Y |  | 5-14, 31-38 |
| A |  | 2, 16 |
| Y | WO 2021/177317 A1 (THINK-LANDS CO., LTD.) 10 September 2021 (2021-09-10) paragraphs [0012]-[0037], fig. 1-11 | 5-14, 31-38 |
| A | CN 112516451 A (GUANGZHOU NALI BIO-TECH CO., LTD.) 19 March 2021 (2021-03-19) entire text, all drawings | 1-38 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **02 February 2024** | **20 February 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

| | International application No. |
|---|---|
| | **PCT/JP2023/043025** |

| Patent document cited in search report | | | Publication date (day/month/year) | Patent family member(s) | | | Publication date (day/month/year) |
|---|---|---|---|---|---|---|---|
| JP | 2017-38781 | A | 23 February 2017 | US 2018/0185624 A1 paragraphs [0024]-[0049], fig. 1-8 CN 107735143 A KR 10-2018-0042162 A WO 2017/030012 A1 | | | |
| WO | 2021/177317 | A1 | 10 September 2021 | (Family: none) | | | |
| CN | 112516451 | A | 19 March 2021 | (Family: none) | | | |

Form PCT/ISA/210 (patent family annex) (July 2022)

**EP 4 628 142 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007061972 A2
- WO 2021177317 A
- JP 2020096790 A